Europäisches Patentamt

European Patent Office

Office européen des brevets

(11) Publication number: 0 287 284
A1

# EUROPEAN PATENT APPLICATION

(21) Application number: 88303143.7

(22) Date of filing: 08.04.88

(51) Int. Cl.4: **A01G 7/00** , **A01G 9/24** , **A01G 31/02**

(30) Priority: 08.04.87 GB 8708441

(43) Date of publication of application:
**19.10.88 Bulletin 88/42**

(84) Designated Contracting States:
**AT BE CH DE ES FR GB GR IT LI LU NL SE**

(71) Applicant: **BAUMGARTNER PAPIERS S.A.**
**Post Box**
**CH-1001 Lausanne(CH)**

(72) Inventor: **Hodds, Harry**
**Northern House 15 Cliff Street**
**Bridlington North Humberside, YO15**
**2NJ(GB)**
Inventor: **Hill, Ronald Poppleton**
**36, Middle Howe Green Manor Park**
**Walkington Beverley, HU17 8TE(GB)**
Inventor: **Demaurex, Louis**
**Mainnesses**
**CH-1302 Vufflens-La-Ville(CH)**
Inventor: **Bonnet, Jacques**
**Chemin de Villard 21**
**CH-1007 Lausanne(CH)**
Inventor: **Veluz, Serge**
**La Repentance**
**CH-1111 Echichens(CH)**

(74) Representative: **McCall, John Douglas et al**
**H. L. Cottrell & Co. Kings Building South**
**Church Side**
**Hull North Humberside, HU1 1RR(GB)**

(54) A sterile propagation system.

(57) A sterile culture medium and delivery/culture vessel for the micropropagation of plants and cell cultures. The culture medium comprises a plug (10) of water absorbant fibrous material, such as cellulose, and a retaining sleeve of, for example, paper or chemical coating which is open at one or both ends. Piror to and during micropropagation the plugs (10) are contained in a delivery/culture vessel which comprises a base tray (1) and a cover (2) which forms a sealable fit over the base tray (1).

Fig 1.

# A STERILE PROPAGATION SYSTEM

The present invention relates to a sterile propagation system, particularly, but not exclusively, intended for the micropropagation of plants. In this respect, a first aspect of the present invention provides a culture medium for explants, a second aspect of the present invention provides a culture vessel in which the explants can be grown on, and a third aspect of the present invention provides a culture cabinet for a plurality of such culture vessels.

Micropropagation is a method for the vegetative propagation of plants under conditions which permit rapid multiplication. In essence "explants" comprising a portion of the shoots or roots of a "mother" plant are excised and freed from bacterial, fungal and sometimes viral contamination. The "explants" are then inoculated under sterile conditions on to a sterile nutrient culture medium. The ideal composition of the culture medium varies according to the plant variety.

In order to maintain sterility, the culture medium and the plant material are enclosed in a sterile culture vessel. After a period of growth the plant material is sub-divided under sterile conditions and re-cultured on fresh culture medium in order to achieve multiplication.

When a sufficient number of "plantlets" have been generated, they are prepared for transfer to the more demanding environment into which they will eventually be planted out e.g. the greenhouse or field. High levels of humidity in the culture vessel affect the condition of the foliage which is subject to rapid desiccation on exposure to low humidity. Considerations in preparing plants for transfer therefore include the preparation of foliage to resist desiccation and the induction of the roots, which may not be present during the multiplication phase.

Plants are prepared for transfer to soil conditions by a period of acclimatisation outside the culture vessel when humidity is reduced, often by gradual stages to lower levels than in the culture vessel.

Due to the fact that all of the plants are derived from the one mother plant,they are all clones of the mother plant and genetically identical to each other. This can be of considerable important with many cash crop plants, trees and shrubs where it is necessary to ensure that each plant retains the mother plants characteristics and where production of uniform seeds may be expensive or impractical. Obviously, plants grown from seed are not clones of the parent plants having been produced through sexual reproduction, and in the case of some hybrid plants the seeds are produced in such low numbers that plants grown from seed are unacceptably expensive. Even when seeds are available germination rates can be low and erratic, and the plants may take a long time to grow to a size at which they can be safely planted out. Put simply then micropropagation allows large numbers of plants to be cloned from a single mother plant and grown on quickly. Plants upon which the technique is commonly used include many agricultural, forestry and ornamental species. The technique is applicable to hard and soft stemmed plants.

When explants are first cut from the mother plant they are particularly vulnerable to pests, moulds, bacteria and viruses, and dehydration is also a very real problem. In order to maintain their sterile condition the plantlets are sub cultured in a sterile environment and then planted in trays filled with a sterile agar gel preparation. These trays are then placed in a culture room or cabinet where the explants are allowed to grow on and develop roots and foliage. The agar solidified medium serves dual roles in that it provides both nutrients and support for the plantlets. Unfortunately, the agar is not ideally suited to either of these roles and suffers from a number of positive disadvantages when it comes to micropropagation.

In the first instance, agar preparations are rarely the same from one batch to another, giving rise to differential growth rates for plantlets. In addition, because the agar gel is a solid growth medium it is not particularly convenient to apply additional water and selected nutrients to the explants. Thus, even though it may be possible to recognise that the agar gel is low in a particular nutrient, through deficiency symptoms displayed by the plantlets, it may not be possible to rectify the problem. Moreover, it has been shown that agar gel does not promote good root development. It is a matter for speculation why this should be so, but possibly it is due to the low level of oxygen in the agar gel or that the soft, non-abrasive nature of the agar gel does not provide the right signals to the root tip to encourage root growth and the development of root hairs. In any event plantlets transferred from the agar gel do not always have a sufficiently vigorous root system to ensure survival.

As the explants are very susceptible to infection in the early stages of their development and the agar gel represents an ideal culture medium for many infectious organisms, great care must be taken to sterilise the agar gel before use. Generally, this means heating the gel and tray in an autoclave. Whilst this sterilisation technique is effective it is not especially convenient.

A further disadvantage associated with the use of agar gel as a culture medium for explants is encountered when the explant has developed roots and foliage, and is ready to be transplanted into a compost for growing on. The plantlet must be removed from the agar gel and the agar gel washed off from the roots. The roots of the plantlet must then be inserted in the compost. This entails handling of the plantlet and even when great care is exercised some of them are damaged. Many of the plantlets do not survive transplantation and mortality of plantlets at this stage is often significant. Roots at this stage are bare, unsupported and easily damaged.

It is an object of the present invention to provide a culture medium which is especially, although not exclusively, intended for growing on explants in which the disadvantages associated with agar gel culture mediums are obviated or substantially mitigated. In particular, it is an object of the present invention to provide a culture medium which can be transferred with the plantlet to compost and which obviates the need to handle the plantlet itself, thus avoiding root disturbance.

It is a further object of the present invention to provide a delivery/culture vessel in which the sterility of the growth medium can be maintained and the explants grown on to plantlets in a sterile and controlled environment.

It is yet another object of the present invention to provide a culture cabinet in which a plurality of culture vessels can be maintained.

According to a first aspect of the present invention there is provided a sterile culture medium for the micropropagation of plants and cell cultures comprising a plug of water absorbant fibrous material and a retaining sleeve or chemical coating which is open at one or both ends. The sleeve or coating may be continuous or perforated.

The plug may be comprised of polyester, acetate or other natural or man-made fibres, but is preferably comprised of cellulose which has the advantage of being biodegradeable. The retaining sleeve may be comprised of polyethlene, polypropylene or other similar materials, but is preferably comprised of paper which, like the cellulose plug, is biodegradable.

The plug may be impregnated with nutrients prior to use, or nutrients can be supplied in aqueous solution to be absorbed by the plug when in use. The plugs do not form a suitable culture medium until moistened which minimises the risk of their carrying infections to the explant or cell cultures to be grown on them. Moreover, they can be readily sterilised prior to use by ionising radiation, chemical or other means.

Unlike agar gel each plug provides a sufficiently solid, rigid support upon and in which plantlets or cell culture can be grown. It has been found that the root system of plantlets grown in such plugs are stronger and more vigorous than those grown in agar gel. After the plantlet has completed its early development it can be transferred easily and without damage to a new environment without removing it from the plug and handling of the plantlet is avoided altogether. The plug will either biodegrade over a period of time or, where the materials from which it is comprised are not readily biodegradeable, the root system of the plantlet will simply grow through it. This enhances the plantlets chances of survival as there is little likelihood of it being damaged in the transfer and trauma is reduced because the roots continue to grow in the plug to which they have become accustomed.

Cell cultures too may benefit from being grown in the culture medium of the present invention, as opposed to the conventional medium of agar gel. The additional support provided by the fibrous material comprising the plugs is expected to enhance growth and allows the cell culture to grow in three dimensions.

According to a second aspect of the present invention there is provided a delivery/culture vessel comprising a base tray, a cover which forms a sealable fit over the base tray and one or more culture mediums in accordance with the first aspect of the present invention contained therein.

Conveniently, the base tray is relatively shallow and the cover is relatively deep. Where the culture vessel is used for micropropagation of plants this allows the plantlets room to grow upwards.

The base tray may be provided with drainage holes through which water, or water with nutrients in solution can be drawn into the culture vessel, or alternatively to allow excess moisture to be drained from the culture vessel. Prior to use these drainage holes are sealed to prevent contamination of the interior of the culture vessel. In order to further facilitate drainage from the culture vessel the bottom of the base tray may be ribbed to raise the drainage holes above the surface on which the culture vessel rests.

An absorbant mat may be provided in the base tray on which the culture medium rests. This absorbent mat provides a reservoir of water and/or nutrients, and improves distribution to each of the culture mediums and minimises the possibility of the culture vessel drying out. This absorbent mat may be impregnated with nutrients to provide the slow release of nutrients to the culture medium. The mat may be ribbed to facilitate drainage through the drainage holes.

The cover may be provided with closeable ventilation holes in it. Alternatively, it may comprise a micro-mesh screen, microporous membrane, or any file or material which allows the flow of air between the interior and exterior of the culture vessel, whilst preventing bacteria, spores and the like from entering the culture vessel. The micro-mesh screen or the like also allows the evaporation of moisture from the culture vessel and, therefore, means for regulating the humidity within the culture vessel may be provided. Conveniently, this comprises a detachable lid which fits over the micro-mesh screen or the like. Closeable apertures may be provided in the lid to further facilitate ventilation and regulation of the humidity within the culture vessel.

Where ventilation is restricted a chemical filter, such as an activated charcoal filter, may be provided inside the culture vessel to prevent the build up of noxious gases.

In one embodiment of the present invention, prior to use the detachable lid may be fitted over the base tray so as to retain the culture mediums in position, the underside of the lid being in close proximity to or in contact with the upper ends of the culture mediums. In this instance the detachable lid makes a sealable fit with the base tray. This embodiment is particularly important in transit to conserve space.

In this embodiment the base tray and the lid may be transported separately from the cover to enable maximum effective use of the space to be made. In this respect the lid ensures that the interior of the tray is kept sterile whilst not adding appreciably to its overall dimensions. The covers can be transported in a stack and advantageously the interior of each is kept sterile by its neighbour which forms a sealable fit inside it. Only the uppermost cover is unprotected and this can be easily remedied by covering it with a disposable lid or film. Of course in this embodiment the detachable lid is provided not only to keep the culture mediums in position in the base tray, but also to cover the micro-mesh film or screen in the top of the cover. The base trays may similarly be transported in a stack.

Preferably, the cover and lid are comprised of a transparent material so as to allow light to be admitted. Conveniently, both the base tray and the cover are comprised of a heat resistant plastics material which allows the culture vessel to be sterilised by autoclaving. However, this is not an essential requirement as the base tray and cover may equally be sterilised by radiation.

In some situations it may not be necessary to illuminate the interior of the culture vessel in which case the base tray and cover may be comprised of non-transparent materials.

In use, the culture vessel is opened in a sterile area, such as a laminar flow cupboard, and where appropriate the detachable lid is removed to uncover the culture mediums. Where the culture mediums and/or the absorbent mat are already impregnated with nutrient, sterile water only is added to each culture medium, otherwise a sterile nutrient solution is added. An explant or a sample of cell culture is then placed in or on the open end of the culture medium.

The cover is then placed over the base tray to enclose and seal the culture vessel which is then placed in an incubation area with appropriate conditions of light and temperature. The detachable lid may be used at this stage to cover the micro-mesh screen. During incubation additional water and nutrients can be introduced into the culture vessel, or excess water drained from the culture vessel through the drainage holes in the base tray.

Once the plantlets or cell cultures have developed sufficiently, the culture vessel is removed from the incubation area. In the case of plantlets the culture vessel may be transferred to an acclimatisation area where the plantlets can become accustomed to the more rigorous and demanding conditions of a less controlled environment before being potted on into a compost. However, it has been found that plantlets grown according to the present invention will in some cases, develop sufficient foliage and a strong enough root system as to make weaning (acclimatisation) un-necessary. As previously indicated the plantlet can be potted on in the growth medium and, therefore, there is no risk of it being damaged or traumatised by removal from the culture medium.

At this stage plantlets may be divided to multiply the number of explants.

According to a third aspect of the present invention there is provided a culture cabinet for a plurality of culture vessels in accordance with the second aspect of the present invention, comprising a cabinet, shelves for supporting said culture vessels, lighting for illuminating the culture vessels, temperature control means for regulating the temperature within the culture cabinet and means for controlling the gas composition within the interior of the cabinet.

Preferably, the shelves for supporting the said culture vessels are mounted on rollers to facilitate withdrawal from the cabinet and examination of the culture vessels.

An embodiment of the present invention will now be described, by way of example, with reference to the accompanying drawings, in which:

Fig. 1 is a perspective view of the delivery/culture vessel according to a first embodiment of the second aspect of the present invention;

Fig. 2 is a perspective view of the delivery/culture vessel shown in Fig. 1 with the cover removed;

4

Fig. 3 is a perspective view of the delivery/culture vessel shown in Fig. 1 with a partially shown lid over the top of the cover of the culture vessel;

Fig. 4 is a side view of a delivery/culture vessel according to a second embodiment of the second aspect of the present invention prior to removal of the lid and positioning of the cover;

Fig. 5 is an exploded side view of the delivery/culture vessel of Fig. 4 with the cover and lid in the relative positions they adopt in use; and

Fig. 6 shows a schematic view of a culture cabinet in accordance with the third aspect of the present invention.

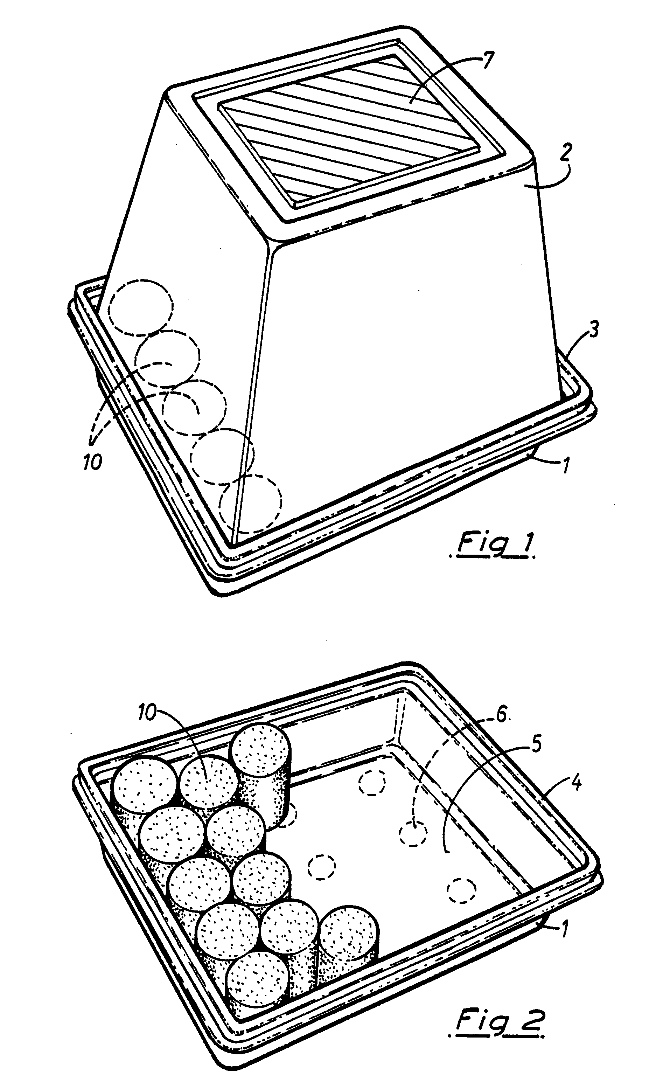

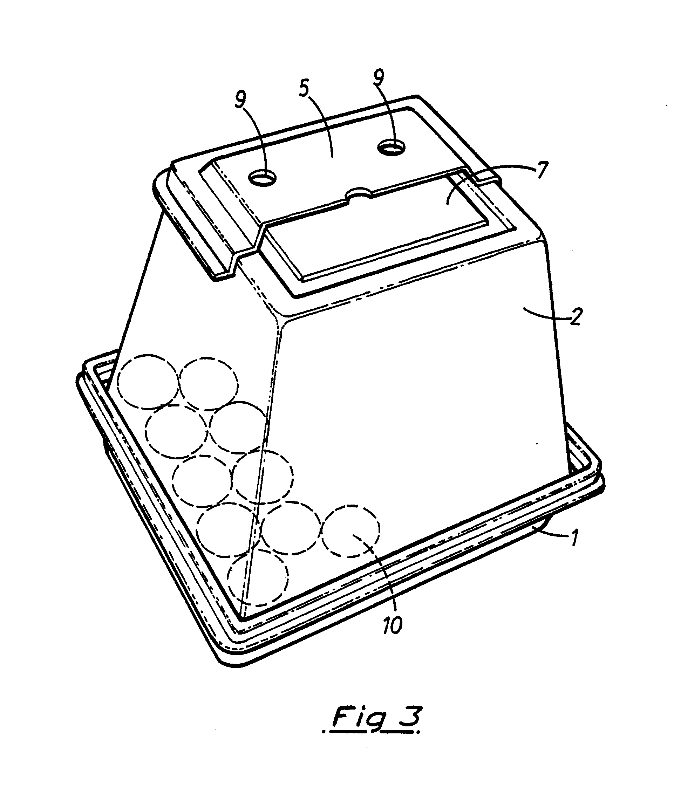

Referring to Fig. 1 of the accompanying drawings there is shown a delivery/culture vessel comprising a shallow base tray 1 and a deep cover 2. Around the bottom of the cover 2 there is a contoured lip 3 which forms a sealable fit over a correspondingly contoured lip 4 (see Fig. 2) around the top of the base tray 1. The floor of the base tray 1 is covered with an absorbant pad 5 and in the bottom of the base tray 1 itself there are provided a plurality of drainage holes 6 which are sealed prior to use with a tear-off strip (not visible). In the top of the cover 2 there is provided a micro-mesh screen 7 which allows air or gases to pass between the interior and exterior of the culture vessel but excludes micro-organisms. As can be seen in Fig. 3 a detachable lid 8 fits over the top of the cover 2 to cover the micro-mesh screen 7. This detachable lid 8 has two closeable ventilation holes 9 in it. As the micro-mesh screen 7 allows the evaporation of water from inside the culture vessel the detachable lid is provided to control this evaporation, and the holes 9 allow ventilation to continue with the lid in position.

Within the base tray 1 there are provided a plurality of culture mediums 10 in the form of cylindrical plugs enclosed in a retaining sleeve which is open at both ends. Each plug is comprised of water absorbant fibrous cellulose material and the retaining sleeve is comprised of paper. As such the whole culture medium is bio-degradable.

The plug may be impregnated with nutrients prior to use, or a solution of nutrients can be added to the plug immediately prior to use. Alternatively, the absorbent pad 5 on the floor of the base tray 1 can be impregnated with slow release nutrients which are absorbed by the culture mediums when water is added and held in the absorbant pad 5.

In use, the cover 2 of the culture vessel is opened in a sterile area, such as a laminar flow cupboard and sterile water or a sterile nutrient solution is added to each culture medium 10, or to the absorbant pad. Then an explant or a sample of cell culture, prepared in accordance with conventional preparation techniques is placed in or on the open end of the culture medium 10.

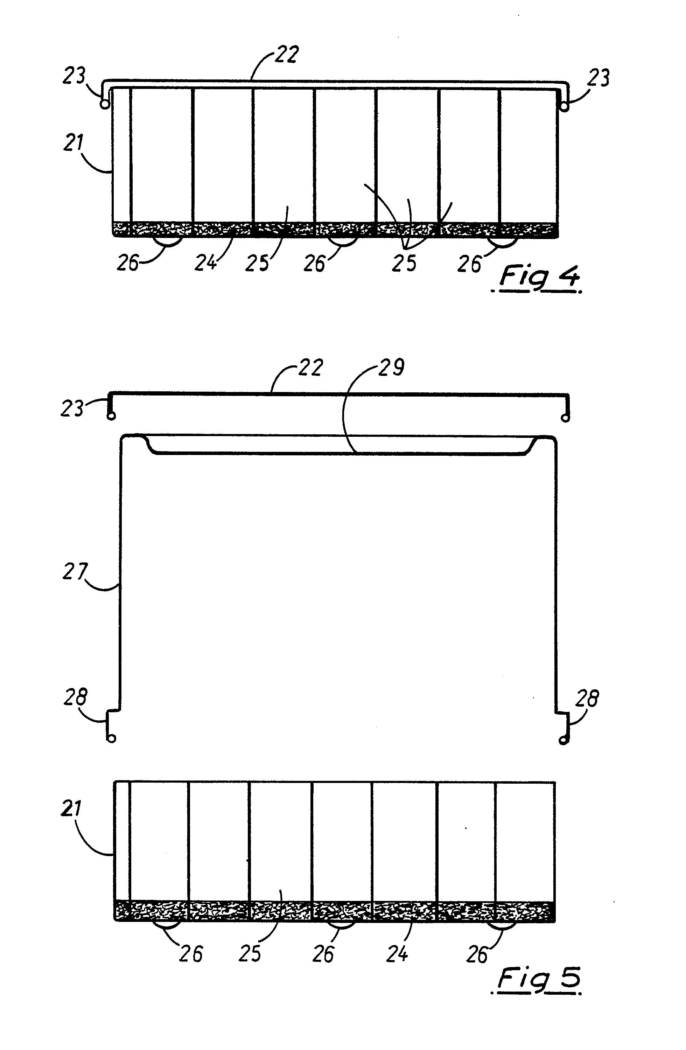

The cover 2 is then replaced over the base tray 1 and the two contoured lips 3 and 4 pressed together to enclose and seal the culture vessel and the detachable lid placed over the screen. The culture vessel is then placed in an incubation area of culture cabinet (see Fig. 4) with appropriate conditions of light, temperature and atmosphere composition. During incubation additional water and nutrients can be introduced into the culture vessel and excess water drained from the culture vessel through the drainage holes 6 in the base tray 1.

Once the explants or cell culture have developed sufficiently, the culture vessel is removed from the incubation area or culture cabinet. In the case of plantlets the culture vessel may be transferred to an acclimatisation area where the plantlets can become accustomed to the more rigorous and demanding conditions of a less controlled environment before being potted on into a compost. With some plantlets this weaning or acclimatisation stage may be omitted as the plantlets will have developed sufficient foliage and a robust enough root system to enable them to be planted straight out. As previously indicated the plantlet can be potted on without removal from the culture medium 10 and, therefore, there is no risk of it being damaged or traumatised by removal.

Unlike agar gel each culture medium 10 provides a sufficiently solid, rigid support upon or in which explants or cell cultures can be grown. Indeed, it has been shown that the root system of plantlets grown in the culture medium 10 are stronger and more vigorous than those grown in agar gel. After the plantlet has completed its early development it can be transferred to a new environment without removal from the culture medium 10 and handling of the plantlet is avoided altogether. The culture medium 10 will biodegrade over a period of time, but if this takes place at a slower rate than the growth of the plantlet this will not inhibit growth as the roots will simply force a way through the enclosing sleeve or through the base of the culture medium.

Cell cultures too may benefit from being grown in the culture medium 10, as opposed to the conventional medium of agar gel. The additional support provided by the absorbant fibrous cellulose material comprising the plug is expected to enhance growth and allows the cell culture to grow in three dimensions.

Referring to Fig. 4 there is shown a transparent plastics tray 21 and transparent plastics lid 22 which closes the open top of the tray 21. A flange 23 extends around the perimeter of the lid 22 and ensures a sealed connection between the lid 22 and the tray 21. An absorbant mat 24 is placed in the bottom of the tray 21 and on this a number of cylindrical absorbant plugs (25) are placed vertically and in close contact with their neighbouring plugs 25.

Drainage holes (not visible) are provided through the absorbant mat 24 and the base of the tray 21 so that the holes through each are made to coincide. Prior to use these drainage holes are sealed by a tear-off strip (not shown). The absorbant mat 24 provides a reservoir of water and may be impregnated with nutrients which are slowly dissolved and absorbed by the plugs 25.

Studs or ribs 26 are formed in the base of the tray 21 so that the tray 21 is raised above any work surface on which it might be placed. These enable surplus liquid to drain more readily through the drainage holes when unsealed.

Referring now to Fig.5 in use, the lid 22 is removed from the tray 21, in a sterile environment such as a laminar airflow cabinet and an explant is placed on the upper surface of each plug 25. It is not actually necessary to handle the plugs 25, but in any event sterile conditions are maintained by the cabinet.

Once the explants have been planted a transparent plastics cover 27 is placed over the top of the tray 21 so as to make a seal between the two, this combination forming a culture vessel for the explants. The cover 27 is sufficiently deep to allow space above the absorbent plugs 26 in which the foliage of the plantlet can grow. A flange 28 is provided around the perimeter of the cover 27 so as to position and seal it on the tray 21. The cover 27 is provided in the top with a micro-mesh screen, microporous membrane, or any film or material 29 which allows the flow of air between the interior and exterior of the culture vessel, whilst preventing bacteria, spores and the like from entering. The micro-mesh screen or the like also allows the evaporation of moisture from the culture vessel. In order to regulate the humidity within the culture vessel the lid 22, previously used during delivery of the tray to its place of use, can be fitted over the top of the cover 27 to close off the micro-mesh screen 29. Closeable apertures (not visible) are provided in the lid 22 to further facilitate ventilation and regulation of the humidity within the culture vessel.

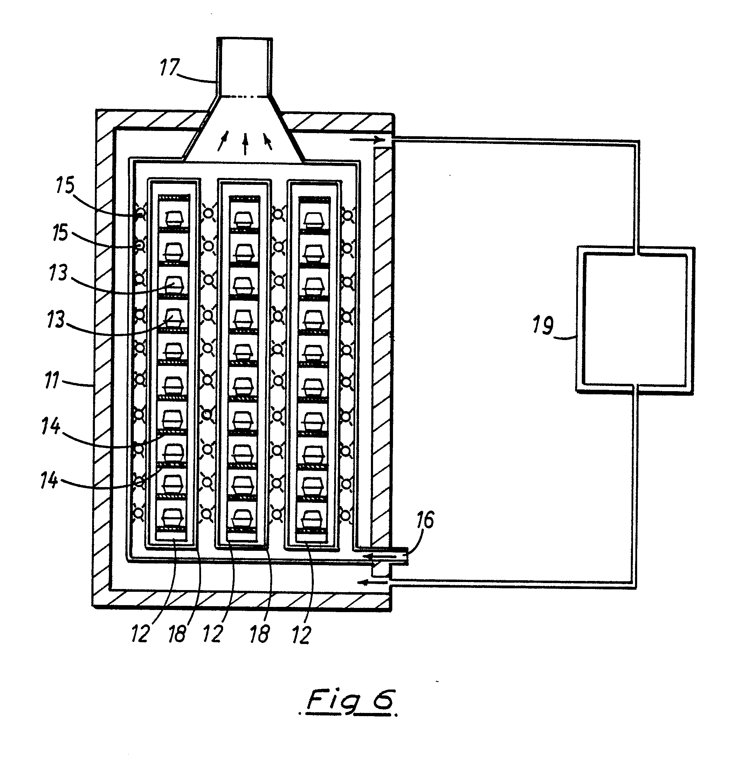

Referring now to Fig. 6 of the accompanying drawings there is shown a schematic view of a culture cabinet in which a plurality of culture vessels according to the second aspect of the present invention can be placed. The culture cabinet comprises a cabinet 11 which is normally closed by a door (not shown). Within the cabinet 11 are three racks of shelves 12 which are mounted on rollers (not shown) to facilitate removal of each rack from the culture cabinet and examination of the culture vessels 13 supported thereon. On each side of each shelf 14 there is provided a strip lamp 15 to illuminate the culture vessels and promote the growth of light dependent explants or cell cultures contained therein.

The lamps 15 generate a great deal of heat inside the cabinet and therefore temperature regulation entails cooling the interior of the cabinet, rather than heating it. To this end ambient air is passed over the lamps 15, coming in through inlet pipe 16 and leaving through outlet pipe 17. The ambient air is separated from those areas of the cabinet in which the racks of shelves 12 are situated by means of transparent dividing walls 18 behind which the lamps are also situated.

The atmosphere within the rack areas is regulated by a control unit 19. As well as sampling the composition of the atmosphere within the rack areas, control unit 19 also regulates its composition. In this respect it will be understood that plants require carbon dioxide to promote the process of photosynthesis and growth, whilst certain hydrocarbon gases such as methane, ethane and ethylene inhibit growth. By introducing these gases into the rack areas at controlled levels it is possible to promote or retard the development of foliage or roots selectively at different stages in the plantlets growth.

## Claims

1. A sterile culture medium for the micropropagation of plants and cell cultures comprising a plug of water absorbant fibrous material and a retaining sleeve or chemical coating which is open at one or both ends.

2. A sterile culture medium according to claim 1, wherein the plug is comprised of cellulose and the retaining sleeve is comprised of paper.

3. A sterile culture medium according to claim 1, or 2, wherein the plug is impregnated with nutrients prior to use.

4. A sterile culture medium according to any preceding claim wherein the sleeve is perforated or in the form of a mesh.

5. A delivery/culture vessel for one or more sterile culture mediums according to any of claims 1 to 4 comprising a base tray and a cover which forms a sealable fit over the base tray.

6. A delivery/culture vessel according to claim 5, wherein the base tray is provided with drainage holes.

7. A delivery/culture vessel according to claim 6, wherein the bottom of the base tray is ribbed to raise the drainage holes above the surface on which the culture vessel rests.

8. A delivery/culture vessel according to any of claims 5, 6 or 7 wherein an absorbant mat is provided in the base tray.

9. A delivery/culture vessel according to claim 8, wherein the absorbant mat is impregnated with nutrients.

10. A delivery/culture vessel according to any one of claims 5 to 9, wherein the cover comprises closeable ventilation holes

11. A delivery/culture vessel according to any one of claims 5 to 9 wherein the cover comprises an air permeable filter which allows the flow of air between the interior and exterior of the culture vessel, whilst preventing bacteria, spores and the like from entering the culture vessel.

12. A delivery/culture vessel according to claim 11, wherein a detachable lid is provided which fits over the air permeable filter and regulates the humidity within the culture vessel.

13. A delivery/culture vessel according to claim 12 wherein the lid closes the open top of the base tray prior to use, thereby enabling the base tray and the cover to be stored, transported separately.

14. A culture vessel according to any one of claims 5 to 13, wherein a chemical filter, such as an activated charcoal filter, is provided within the culture vessel to prevent the build up of noxious gases.

15. A culture cabinet for a plurality of culture vessels in accordance with any of claims 5 to 14 comprising a cabinet, shelves for supporting said culture vessels, lighting for illuminating the culture vessels, temperature control means for regulating the temperature within the culture cabinet and means for controlling the gas composition within the interior of the cabinet.

_Fig 1_

_Fig 2_

_Fig 3_

*Fig 4.*

*Fig 5.*

_Fig 6._

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 117 766  (CREATIONS CHALLET HERAUT S.A.R.L.) <br> * complete document * | 1-4 | A 01 G  7/00 <br> A 01 G  9/24 <br> A 01 G  31/02 |
| Y | | 5-9 | |
| X | DE-C-1 136 150  (A. KNEPPER) <br> * complete document * | 1-4 | |
| Y | | 5-9 | |
| Y | FR-A-1 551 807  (U. BONISTALLI) <br> * complete document * | 5-9 | |
| A | DE-B-1 944 539  (CONTROLLED ENVIRONMENTS LTD.) <br> * complete document * | 10-12, 15 | |

TECHNICAL FIELDS
SEARCHED (Int. Cl.4)

A 01 G  7/00
A 01 G  9/00
A 01 G  31/00

The present search report has been drawn up for all claims

| Place of search | Date of completion of the search | Examiner |
|---|---|---|
| BERLIN | 05-07-1988 | WUNDERLICH J E |

EPO FORM 1503 03.82 (P0401)